# EUROPÄISCHE PATENTSCHRIFT

(11) **EP 2 119 409 B1**
(45) Veröffentlichungstag und Bekanntmachung des Hinweises auf die Patenterteilung: **10.10.2012**
(21) Anmeldenummer: 08156279.5
(22) Anmeldetag: 15.05.2008
(51) Int. Cl.: A61B 19/00

(54) **Gelenk-Rekonstruktionsplanung mit Modelldaten**
Joint reconstruction plan with model data
Planification de la reconstruction de l'articulation à l'aide de données de modèle

(43) Veröffentlichungstag der Anmeldung: 18.11.2009
(73) Patentinhaber: BrainLAB AG, 85622 Feldkirchen (DE)
(72) Erfinder: Haimerl, Martin, 82205, Gilching (DE); Schubert, Mario, 85586, Poing (DE)
(74) Vertreter: Schwabe - Sandmair - Marx

(56) Entgegenhaltungen:
- EP-A- 1 693 798
- WO-A-00/63844
- US-A- 5 871 018
- US-A1- 2005 267 353

## Beschreibung

Die Erfindung betrifft das Gebiet der Gelenk-Rekonstruktionsplanung, insbesondere betrifft sie ein computergestütztes Planungsverfahren für die Rekonstruktion von Formveränderungen an Gelenkknochen. Gelenkknochen-Formveränderungen sind eine mögliche Ursache für arthritische Gelenkerkrankungen. Beispielsweise können im Bereich des Hüftgelenks Knochenanomalien auftreten, die bei der Beinbewegung zu Knochen-Kollisionen im Gelenkbereich führen und - insbesondere auf längere Dauer - Abnutzungserscheinungen hervorrufen. Zur Vorbereitung von Rekonstruktions-Behandlungen, also solchen Behandlungen, welche wieder eine geeignete Knochenform herstellen, ist eine Planung notwendig, die computergestützt erfolgen kann. Geplant wird die Neu-Formung des Femur-Kopfes, manchmal auch des Gelenkpfannen-Randes, in einer solchen Weise, dass unnatürliche Kollisionen zwischen dem Oberschenkelknochen und dem Acetabulum (Gelenkpfanne) in der Gelenkumgebung verhindert werden. Dabei sollte eine natürliche Form für den Femur-Kopf erreicht werden. Die Grundlage solcher Planungen war bisher die Bestimmung der Kontur von Gelenkknochen auf zweidirnensionaler Basis, beispielsweise in einer bestimmten Schnittebene oder Abbildungsebene. Im "Journal of Bone and Joint Surgery", vol. 84 -B, no. 4, May 2002, Seiten 556 bis 560 beschreiben Noetzli et al die Bestimmung der Kontur eines Oberschenkelhalses, und hierfür wird ein Mess- und Winkelsystem angegeben, auf welches auch hierin später Bezug genommen wird.

Das US-Patent 5,871,018 beschreibt ein computergestütztes Planungsverfahren zur Vorbereitung einer Rekonstruktionsbehandlung eines Gelenkknochens, welche an dem Gelenkknochen, der Formveränderungen aufweist, durch Einsetzen eines Implantats wieder eine geeignete Knochenform herstellt.

Es ist die Aufgabe der vorliegenden Erfindung, eine Gelenk-Rekonstruktionsplanung zu ermöglichen, die eine ausreichend genaue Rekonstruktion bei geringem Aufwand ermöglicht. Insbesondere sollen aufwändige und längere Bildverarbeitungstätigkeiten vermieden werden, um die Planung flexibler einsetzbar zu machen.

Diese Aufgabe wird durch ein computergestütztes Planungsverfahren gemäß dem Anspruch 1 gelöst. Die Unteransprüche definieren bevorzugte Ausführungsformen der Erfindung.

Das erfindungsgemäße Verfahren weist die folgenden Schritte auf:
- Ein dreidimensionaler Patientendatensatz wird akquiriert;
- dem Patientendatensatz werden rekonstruktionstypische Modelldaten, insbesondere Modellbilddaten, zugeordnet,
- mittels der Modelldaten werden Resektions-Hilfsbereiche bestimmt; und
- die Resektions-Hilfsbereiche werden zusammen mit dem Patientendatensatz bildlich ausgegeben bzw. visualisiert,
- wobei die Resektionsbereiche aus mindestens einem Teil der Überschneidungen der Resektions-Hilfsbereiche und des akquirierten Gelenkknochen-Patientendatensatzes definiert werden.

Anders ausgedrückt verwendet die vorliegende Erfindung Modelldaten, die sich für die anstehende Knochenrekonstruktion eignen, um durch Verarbeitung solcher Modellinformationen auf die Abrasions- oder Resektionsbereiche schließen zu können. Die Verarbeitung solcher Modelldaten, die Modellbilddaten sein können, und ihre Verknüpfung mit dem Patientendatensatz, insbesondere einem Bilddatensatz, ist in einfacher Weise durchführbar und führt auch zu schnellen Ergebnissen, insbesondere ohne dass der Inhalt eines Patientenbilddatensatzes in aufwändiger Weise analysiert werden muss. Bei solchen Analysen wird nämlich aus dem Bildmaterial auf die Eigenschaften der abgebildeten Elemente, ihre Identität und ihre Abgrenzung zu anderen Elementen geschlossen; einen solchen Vorgang nennt man Segmentierung. Die Segmentierung ist sehr rechenaufwändig und deshalb dauern die entsprechenden Planungsschritte relativ lange. Wenn mit Hilfe der Modelldaten auf Segmentierungsschritte verzichtet werden kann, wird die Planung flexibler einsetzbar und kann auch angepasst und verändert werden. Insbesondere kann auch eine intraoperative Planung mit intraoperativen Bildaufnahmen (CT, MR) stattfinden. Ein weiterer Vorteil der Eliminierung einer Segmentierung liegt darin, dass die Segmentierung oft sehr schwierig wird, wenn Bilddatensätze von Gelenkumgebungen erstellt werden. Für gewöhnlich werden MR-Bilder (Kernspintomographie) erstellt, weil Weichgewebedefekte eine große Rolle bei den in Frage stehenden Erkrankungen spielen. Es ist aber sehr schwierig, Knochenstrukturen gut in MR-Daten zu segmentieren, und auch aus diesem Grund ist die erfindungsgemäße Verwendung von rekonstruktionstypischen Modelldaten zur Bestimmung der Resektions-Hilfsbereiche und schließlich der Resektionsbereiche selbst von großem Vorteil gegenüber dem Stand der Technik.

Als Patientendatensatz kann verwendet werden:
- Ein Bilddatensatz der Gelenkknochenumgebung, der mittels eines medizintechnischen bildgebenden Verfahrens erstellt wird, oder
- ein Oberflächenmodell des Gelenkknochens, das insbesondere durch ein registriertes Werkzeug abgegriffen oder abgetastet wurde (z.B. Pointer- oder Licht/Laserabtastung),
oder eine Kombination hiervon.

Der Patientendatensatz, speziell der Bilddatensatz, kann positionell registriert werden, wobei insbesondere Referenzkoordinatensysteme oder Richtungsinformationen im Datensatz bestimmt bzw. geplant werden. Bei Pointerabtastungen mit einem im Tracking-/Navigationssystem registrierten Pointer ergibt sich die Registrierung des Oberflächenmodells von selbst.

Das bildgebende Verfahren kann ein Computertomographieverfahren, ein Kernspintomographieverfahren oder ein Röntgenverfahren, speziell mit Volumenerfassung sein.

Es ist erfindungsgemäß möglich, die Rekonstruktionsplanung auf einem medizintechnischen Navigationssystem vorzunehmen, dem der Patientendatensatz, speziell der Bilddatensatz, und der Modelldatensatz zur Verfügung gestellt werden. Die Resektionsbereiche können dann auf einer Bildausgabe des Navigationssystems visualisiert werden.

Bei einer Ausführungsform der Erfindung werden als Referenzkoordinatensysteme bzw. als Richtungsinformationen eine oder mehrere der folgenden Informationen geplant bzw. bestimmt: Die Femur-Halsachse, die mechanische Femurachse, der Mittelpunkt des Femur-Kopfes, der Radius der Femur-Kopf-Kugel, die posteriore Kondylenachse, die Epi-Kondylenachse, Beckenebenen, insbesondere die mittsagittale und/oder die vordere Beckenebene, und die Acetabulumebene. Mit nur einigen dieser Informationen lässt sich schon eine Kugel in den Femur-Kopf einbeschreiben, und die Abrasions- bzw. Resektionsbereiche lassen sich zumindest teilweise schon eingrenzen oder definieren.

Die Zuordnung der Modelldaten zu dem Patientendatensatz, speziell dem Bilddatensatz erfolgt insbesondere durch ein computergestütztes Übereinanderlegen bzw. Matching von Elementen der Modelldaten und im Wesentlichen formmäßig übereinstimmenden Elementen aus den Gelenkknochen-Daten bzw. -Bilddaten.

Resektionsbereiche, die sich aus mindestens einem Teil der Überschneidungen der Resektions-Hilfsbereiche und des akquirierten Gelenkknochen-Patientendatensatzes ergeben, können der Rekonstruktionsplanung zugrunde gelegt werden. Bei einer Bestimmung der Resektionsbereiche können auch die Modelldaten selbst als Teilbereich oder ausgeschlossener Teilbereich berücksichtigt werden. In diesem Fall würden also beispielsweise Modelldaten wie sie oben beschrieben wurden (z.B. Radius der Femur-Kopf-Kugel) noch zusätzlich verwendet, um die letztendlich aufzufindenden Resektionsbereiche einzugrenzen.

Die oben genannten Modelldaten können Daten der folgenden Natur umfassen:
- geometrische Grundformen, insbesondere Kugeln, Kugelform-Hohlkörper, Zylinder, Sattelformen oder Kombinationen hiervon,
- Achsen, Flächen, Mittelpunkte, Umfänge und Ränder und deren Punkte oder Flächen;
- Winkelbereiche, insbesondere mit Ursprüngen an vorbestimmten oder charakteristischen Mittel-, Achsen- oder Flächenpunkten, speziell α- und/oder β-Winkel nach Noetzli et al.; und
- standardisierte Referenzdaten für das Gelenk, insbesondere Referenzmodelldaten,
oder aus mehreren der vorgenannten Daten zusammengesetzt sein.

Um die Planung zu optimieren, kann eine Korrektur der Referenzdaten stattfinden, wobei diese Korrektur anhand von gelenk- oder gelenkknochen-typischen Daten erfolgen würde, z. B. Antetorsionswinkel, Retroversionswinkel, CCD-Winkel, vordere/mittsagittale Beckenebenenlage, Beckenneigung. Ferner kann anhand des akquirierten Patientendatensatzes eine automatische Auswahl eines geeigneten Referenzdatenmodells stattfinden.

Das erfindungsgemäße Verfahren kann speziell für ein Hüftgelenk, ein Schultergelenk oder ein Kniegelenk angewendet werden, aber grundsätzlich bei allen Gelenken und Gelenkumgebungen Anwendung finden.

Die Erfindung betrifft ferner gemäß einem weiteren Aspekt ein Programm, das, wenn es auf einem Computer läuft oder in einem Computer geladen ist, den Computer veranlasst, ein Verfahren durchzuführen, wie es oben in den verschiedenen Ausführungsformen erläutert worden ist. Ferner betrifft sie noch ein Computerprogramm-Speichermedium, das ein solches Programm aufweist.

Die Erfindung wird im Weiteren anhand von Ausführungsformen und optionalen Erweiterungen und unter Bezugnahme auf die beiliegenden Zeichnungen näher erläutert. Sie kann alle hierin beschriebenen Merkmale einzeln sowie in jedweder sinnvollen Kombination umfassen. In den Zeichnungen zeigen:
- Fig. 1: eine schematische Darstellung eines Gelenkknochens im Umfeld eines medizintechnischen Trackingsystems; und
- Fig. 2: eine Bildschirmausgabe eines erfindungsgemäßen Planungssystems.

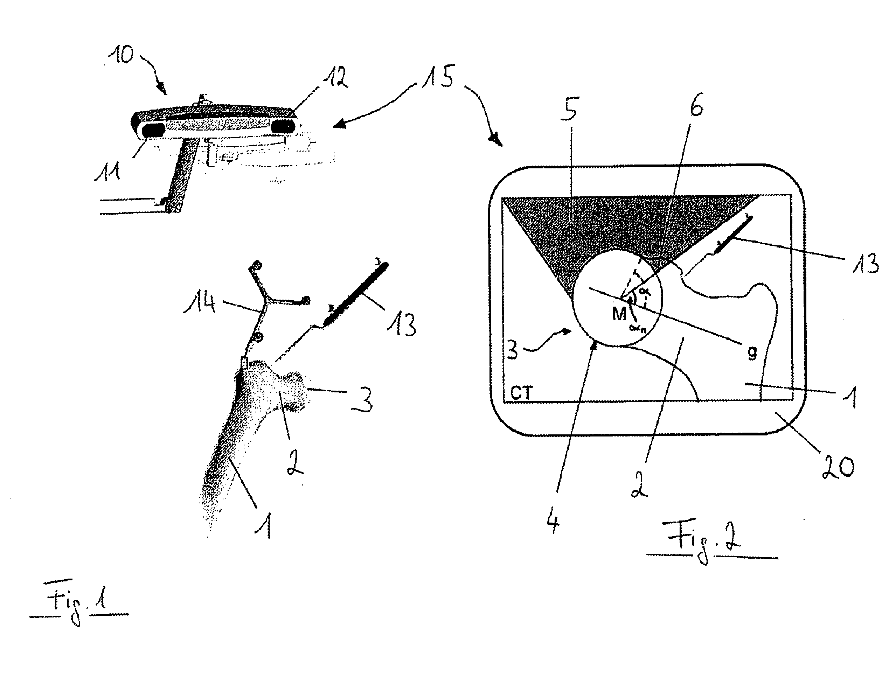

In der Fig. 1 wird schematisch gezeigt, wie ein Oberteil eines Oberschenkelknochens mit Hilfe eines medizintechnischen Trackingsystems 10 positionell geortet und verfolgt bzw. registriert werden kann. Das Trackingsystem 10 weist zwei Kameras 11 und 12 auf, welche die Position von speziellen Markern tracken können. Solche Marker befinden sich beispielsweise an dem Referenzstern 14 oder an dem Pointer 13, die im unteren Teil zusammen mit der Abbildung des Femurknochens 1 dargestellt sind. An letzterem ist der Oberschenkelhals 2 sowie der Femur-Kopf 3 zu erkennen.

Der Referenzstern 14 ist fest mit dem Femur 1 verbunden. Weil vom Femur 1 vorher ein Bilddatensatz erstellt worden ist, kann dieser Bilddatensatz nun über den Referenzstern 14 dem Femurknochen 1 positionell zugeordnet, also der Femurknochen registriert werden. Damit ist dann die Lage spezieller Referenzkoordinaten bekannt und auch Richtungsinformationen sind verfügbar, z.B. über die Lage der Femurachse, der Halsachse oder des Mittelpunktes des Kopfes 3. Mit dem Pointer 13 können spezielle Stellen auf der Oberfläche des Knochens 1 angefahren werden, der Pointer kann hier aber auch stellvertretend für jedes andere navigierbare Instrument (Werkzeug) stehen.

Die Fig. 2 zeigt eine Bildausgabe eines Navigationssystems, mit dem die erfindungsgemäße Planung durchgeführt wird. Schematisch wird das Navigationssystem (mit dem Trackingsystem und der Bildschirmausgabe) in den beiden Zeichnungen mit dem Bezugszeichen 15 angedeutet; die Rechnereinheit ist nicht separat dargestellt, aber üblicherweise vorhanden.

Auf der Bildausgabe 20 sind wiederum in zweidimensionaler Darstellung der Femurknochen 1 und der Oberschenkelhals 2 zu sehen. Der Femur-Kopf 3 ist von der einbeschriebenen Kugel (Kreis in der zweidimensionalen Darstellung) 4 verdeckt. Der Mittelpunkt der Kugel ist mit M bezeichnet und er fällt mit dem Mittelpunkt des Femur-Kopfes zusammen. Die Halsachse trägt die Bezeichnung g. Der Winkel α, der aus dem eingangs genannten Meß- bzw. Winkelsystem nach Noetzli et al. stammt, ist der Winkel zwischen der Halsachse g und dem Radialstrahl, der vom Mittelpunkt M durch den Punkt auf dem Kreis verläuft, an dem die Knochenkontur erstmals von der sphärischen Form abweicht. Der Winkel αₙ bezeichnet dann den Winkel zwischen der Halsachse g und dem Radialstrahl, bei dem am Kreis normalerweise der Knochen vorhanden sein kann. Mit den Winkeln α und αₙ, der Halsachse g, dem Mittelpunkt M und der Kugel (Kreis) lässt sich ein Resektions-Hilfsbereich 5 festlegen und der Resektionsbereich 6 lässt sich aus den Überschneidungen ermitteln, ohne dass der Bilddatensatz, aus dem die Darstellung 20 stammt, segmentiert werden muss.

Bei einer erfindungsgemäßen Ausführungsform werden dazu zunächst Referenzkoordinatensysteme oder Richtungsinformationen im Bilddatensatz geplant, nämlich vorzugsweise die Halsachse g, die mechanische Femur-Achse, der Mittelpunkt des Femur-Kopfes und dessen Kugelradius. Die Referenzierung bzw. Registrierung erfolgt über eine angebrachte Referenz 14. Es kann auch ein CT-Fluoroskopie-Matching mit einem C-Bogen-Röntgengerät vor Ort durchgeführt werden oder jedwede andere Registrierung. Optional kann natürlich jedwede andere Modellinformation mitgeplant oder berücksichtigt werden, insbesondere andere Knochenachsen oder Knochenflächen. Gemäß dem Referenzkoordinatensystem bzw. den Richtungsinformationen ist nun eine Grundplanung nach standardisierten Modellen möglich (z.B. α-Winkel, detailliertere Analyse der gewöhnlichen Knochenanatomien und ihr Verhältnis bei Kollisions- bzw. Stoßproblemen). Die Abrasionsbereiche (Resektionsbereiche) werden aus diesen Informationen definiert und die Konstruktion dieser Flächen basiert auf den Strahlen, die vom Femur-Kopf ausgehen und ihrer Richtung nach durch die standardisierten Modelle definiert werden. In der Fig. 2 definieren diese Strahlen beispielsweise den Resektions-Hilfsbereich 5.

Die Abrasions- oder Resektionsbereiche oder - andersherum ausgedrückt - die Nicht-Abrasionsbereiche können dann in zweidimensionalen Ansichten des Bilddatensatzes visualisiert werden, der voroperativ oder auch intraoperativ aufgenommen wurde. In der Fig. 2 ergibt sich die Abrasionsfläche aus der Schnittmenge zwischen dem Knochen 1 im Bereich 3 und der Resektionshilfsfläche 5, abzüglich der Kreisfläche 4, als Resektionsfläche 6. Im Bereich der Resektionsfläche 6 kann das Knochengewebe entfernt werden, um dem Knochen seinen gewünschten Zustand zu geben.

Wenn diese Resektion bzw. Abrasion erfindungsgemäß geplant ist, kann mit Hilfe der Navigation die eigentliche Operationstätigkeit visuell unterstützt werden.

Im Weiteren werden noch optionale bzw. vorteilhafte Weiterbildungen des erfindungsgemäßen Verfahrens besprochen. So kann beispielsweise eine Datenfusion von voroperativen Datensätzen mit Planungsinformationen (z.B. segmentierten CT- oder MR-Datensätzen) und intraoperativen Daten (z.B. intraoperativ akquirierten MR-Daten) erfolgen. Ferner kann ein Roh-3D-Modell erzeugt werden (z.B. aus einigen wenigen Referenzpunkten oder -achsen/-ebenen, usw.) und für Visualisierungsaufgaben verwendet werden. Eine weitere Option besteht darin, eine grobe Segmentierung als Hilfe für den Navigationsprozess zu verwenden, z.B. für ein CT/MR-Fluoro-Matching oder für eine 3D-Visualisierung des Knochens mit Volume-Rendering-Techniken. Solche groben Segmentierungen können hierzu ausreichen aber trotzdem schnell und flexibel genug einsetzbar sein (im Gegensatz zu den ansonsten verwendeten Detail-Segmentierungen).

Es ist möglich, Korrekturen gemäß unterschiedlichen Ausrichtungen des Femurknochens (Antetorsionswinkel, CCD-Winkel) und des Beckens (z.B. Neigung, Ante/Retroversion des Acetabulums) durchzuführen. Dies ist nützlich, weil einige Standardtechniken sich auf restriktive Annahmen bezüglich der Anatomie verlassen. Beispielsweise zieht der α-Winkel nur die Halsachse in Betracht. Ein Ansatz aber, der auf einem tatsächlichen Bewegungsspielraum basiert, muss die Antetorsion, den CCD-Winkel, die Neigung, usw. ebenfalls berücksichtigen.

Obwohl sie hier im Ausführungsbeispiel anhand eines Femur-Kopfes beschrieben wurde, kann die erfindungsgemäße Navigationsplanung und die darauf basierende Navigation auch auf der "gegenüberliegenden Seite", nämlich auf der Acetabulum- oder Gelenkpfannenseite angewendet werden.

Ferner kann eine Rekonstruktion der lokalen Knochenstruktur-Oberflächen durchgeführt werden, um die Abrasions- bzw. Resektionsbereiche genauer zu definieren. Beispielsweise kann man die Oberfläche der Kopf-Hals-Bindung mit einem Pointer abgreifen und die abgegriffenen Punkte digitalisieren. In der Fig. 2 ist der Pointer 13 bei dieser Tätigkeit dargestellt. Diese Oberfläche kann dann verwendet werden, um das Abrasionsvolumen detailliert zu definieren. Die Information der digitalisierten Punkte und die Bilddatensatz-Informationen können auch kombiniert werden.

## Patentansprüche

1. Computergestütztes Planungsverfahren zur Vorbereitung einer Rekonstruktionsbehandlung eines Gelenkknochens (1, 2, 3), welche an dem Gelenkknochen (1, 2, 3), der Formveränderungen aufweist, durch Resektion von Resektionsbereichen wieder eine geeignete Knochenform herstellt, mit den folgenden Schritten:
- ein dreidimensionaler Gelenkknochen-Patientendatensatz wird akquiriert;
- dem Gelenkknochen-Patientendatensatz werden rekonstruktionstypische Modelldaten, insbesondere Modellbilddaten (g, M, 4, α, αₙ), zugeordnet,
- mittels der Modelldaten werden Resektions-Hilfsbereiche (5) bestimmt; und
- die Resektions-Hilfsbereiche (5) werden zusammen mit dem Patientendatensatz bildlich ausgegeben bzw. visualisiert,
- wobei die Resektionsbereiche (6) aus mindestens einem Teil der Überschneidungen der Resektions-Hilfsbereiche (5) und des akquirierten Gelenkknochen-Patientendatensatzes definiert werden.

2. Verfahren nach Anspruch 1, bei dem als Patientendatensatz verwendet wird:
- ein Bilddatensatz der Gelenkknochenumgebung, der mittels eines medizintechnischen bildgebenden Verfahrens erstellt wird, oder
- ein Oberflächenmodell des Gelenkknochens, das insbesondere durch ein registriertes Werkzeug abgegriffen oder abgetastet wurde,
oder eine Kombination hiervon.

3. Verfahren nach Anspruch 2, bei dem der Patientendatensatz, speziell der Bilddatensatz, positionell registriert wird, wobei insbesondere Referenzkoordinatensysteme oder Richtungsinformationen im Datensatz bestimmt bzw. geplant werden.

4. Verfahren nach einem der Ansprüche 2 oder 3, bei dem das bildgebende Verfahren ein Computertomographieverfahren, ein Kernspintomographieverfahren oder ein Röntgenverfahren, speziell mit Volumenerfassung ist.

5. Verfahren nach einem der Ansprüche 1 bis 4, bei dem die Rekonstruktionsplanung auf einem medizintechnischen Navigationssystem (15) erfolgt, dem der Patientendatensatz, speziell der Bilddatensatz und der Modelldatensatz zur Verfügung gestellt werden.

6. Verfahren nach Anspruch 5, bei dem die Resektionsbereiche auf einer Bildausgabe (20) des Navigationssystems visualisiert werden.

7. Verfahren nach einem der Ansprüche 1 bis 6, bei dem als Referenzkoordinatensysteme bzw. als Richtungsinformationen eine oder mehrere der folgenden Informationen geplant bzw. bestimmt werden: Die Femur-Halsachse (g), die mechanische Femurachse, der Mittelpunkt (M) des Femur-Kopfes (3), der Radius der Femurkopf-Kugel (4), die posteriore Kondylenachse, die Epi-Kondylenachse, Beckenebenen, insbesondere die mittsagittale oder die vordere Beckenebene und die Acetabulumsebene.

8. Verfahren nach einem der Ansprüche 1 bis 7, bei dem die Zuordnung der Modelldaten zu dem Patientendatensatz, speziell dem Bilddatensatz durch ein computergestütztes Übereinanderlegen bzw. Matching von Elementen der Modelldaten und im Wesentlichen formmäßig übereinstimmenden Elementen aus den Gelenkknochen-Daten bzw. -Bilddaten erfolgt.

9. Verfahren nach Anspruch 1, bei dem bei der Bestimmung der Resektionsbereiche (6) auch die Modelldaten (4) selbst als Teilbereich oder ausgeschlossener Teilbereich berücksichtigt werden.

10. Verfahren nach einem der Ansprüche 1 bis 9, bei dem die Modelldaten Daten der folgenden Natur umfassen:
- geometrische Grundformen, insbesondere Kugeln, Kugelform-Hohlkörper, Zylinder, Sattelformen, oder Kombinationen hiervon;
- Achsen, Flächen, Mittelpunkte, Umfänge und Ränder und deren Punkte oder Flächen;
- Winkelbereiche, insbesondere mit Ursprüngen an vorbestimmten oder charakteristischen Mittel-, Achsen- oder Flächenpunkten;
- standardisierte Referenzdaten für das Gelenk, insbesondere Referenzmodelldaten,
oder aus mehreren der vorgenannten Daten zusammengesetzt sind.

11. Verfahren nach Anspruch 10, bei dem eine Korrektur der Referenzdaten anhand von Gelenk- oder Gelenkknochen-typischen Daten, z.B. Antetorsionswinkel, Retroversionswinkel, CCD-Winkel, vordere/ sagittale Beckenebenenlage, Beckenneigung, stattfindet.

12. Verfahren nach Anspruch 10 oder 11, bei dem anhand des akquirierten Patientendatensatzes eine automatische Auswahl eines geeigneten Referenzdatenmodells stattfindet.

13. Verfahren nach einem der Ansprüche 1 bis 12, das für ein Hüftgelenk, ein Schultergelenk oder ein Kniegelenk angewendet wird.

14. Programm, das, wenn es auf einem Computer läuft oder in einem Computer geladen ist, den Computer veranlasst, ein Verfahren gemäß einem der Ansprüche 1 bis 13 durchzuführen.

15. Computerprogramm-Speichermedium, das ein Programm nach Anspruch 14 aufweist.

## Claims

1. A computer-assisted planning method for preparing a reconstruction treatment of a joint bone (1, 2, 3) which restores a suitable shape of the bone on the joint bone (1, 2, 3) which exhibits changes in shape by resecting resection regions, comprising the following steps:
- a three-dimensional patient data set of the joint bone is acquired;
- reconstruction-type model data, in particular model image data (g, M, 4, α, αₙ), are assigned to the patient data set of the joint bone;
- resection auxiliary regions (5) are determined by means of the model data; and
- the resection auxiliary regions (5) are visually outputted and/or visualised together with the patient data set,
- wherein the resection regions (6) are defined from at least some of the impingements between the resection auxiliary regions (5) and the acquired patient data set of the joint bone.

2. The method according to Claim 1, wherein the patient data set used is:
- an image data set of the joint bone environment, which is produced by means of a medical imaging method; or
- a surface model of the joint bone, which has in particular been tapped or scanned using a registered tool;
or a combination of these.

3. The method according to Claim 2, wherein the patient data set, specifically the image data set, is positionally registered, wherein reference co-ordinate systems or direction information are in particular determined and/or planned in the data set.

4. The method according to any one of Claims 2 or 3, wherein the imaging method is a computed tomography method, a magnetic resonance tomography method or an x-ray method, specifically with volume detection.

5. The method according to any one of Claims 1 to 4, wherein the reconstruction is planned on a medical navigation system (15) which is provided with the patient data set, specifically the image data set, and the model data set.

6. The method according to Claim 5, wherein the resection regions are visualised on an image output (20) of the navigation system.

7. The method according to any one of Claims 1 to 6, wherein one or more of the following items of information are planned and/or determined as the reference co-ordinate systems and/or direction information: the femoral neck axis (g); the mechanical femoral axis; the centre point (M) of the femoral head (3); the radius of the sphere (4) of the femoral head; the posterior condylar axis; the epicondylar axis; pelvic planes, in particular the mid-sagittal or front pelvic plane and the acetabular plane.

8. The method according to any one of Claims 1 to 7, wherein the model data are assigned to the patient data set, specifically to the image data set, by computer-assisted superimposing and/or by matching elements of the model data and elements from the joint bone data and/or joint bone image data which substantially correspond in terms of their shape.

9. The method according to Claim 1, wherein when determining the resection regions (6), the model data (4) themselves are also taken into account as a partial region or excluded partial region.

10. The method according to any one of Claims 1 to 9, wherein the model data include data of the following nature:
- basic geometric shapes, in particular spheres, spherical hollow bodies, cylinders, saddle shapes or combinations of these;
- axes, areas, centre points, circumferences and edges, and points or areas of them;
- angular ranges, in particular having their origins at predetermined or characteristic centre points, axial points or surface points;
- standardised reference data for the joint, in particular reference model data;
or are composed of several of the aforesaid data.

11. The method according to Claim 10, wherein the reference data are corrected on the basis of joint-type or joint bone-type data, for example antetorsion angles, retroversion angles, CCD angles, location of the front/sagittal pelvic plane, or pelvic inclination.

12. The method according to Claim 10 or 11, wherein a suitable reference data model is automatically selected on the basis of the acquired patient data set.

13. The method according to any one of Claims 1 to 12, which is used for a hip joint, a shoulder joint or a knee joint.

14. A program which, when it is running on a computer or is loaded on a computer, causes the computer to perform a method in accordance with any one of Claims 1 to 13.

15. A computer program storage medium which comprises a program according to Claim 14.

## Revendications

1. Procédé de planification assisté par ordinateur destiné à préparer un traitement de reconstruction d'un os d'articulation (1, 2, 3), lequel traitement reconstruit une forme d'os adaptée sur l'os d'articulation (1, 2, 3) qui présente des changements de forme, par résection de régions de résection, comportant les étapes suivantes consistant à :
- acquérir un ensemble de données de patient tridimensionnelles concernant un os d'articulation,
- attribuer des données de modèle typiques d'une reconstruction, en particulier des données d'image de modèle (g, M, 4, α, αₙ) à l'ensemble de données de patient concernant un os d'articulation,
- déterminer des régions auxiliaires de résection (5) au moyen des données de modèle, et
- générer en images ou visualiser les régions auxiliaires de résection (5) en association avec l'ensemble de données de patient,
- dans lequel les régions de résection (6) sont définies à partir d'au moins une partie des intersections des régions auxiliaires de résection (5) et de l'ensemble acquis de données de patient concernant un os d'articulation.

2. Procédé selon la revendication 1, dans lequel l'ensemble de données de patient utilisé est :
- un ensemble de données d'image de l'environnement de l'os d'articulation, qui a été généré au moyen d'un procédé d'imagerie médicale, ou
- un modèle de surface de l'os d'articulation qui a en particulier été prélevé ou balayé par un outil enregistré,
ou une combinaison de ceux-ci.

3. Procédé selon la revendication 2, dans lequel l'ensemble de données de patient, en particulier l'ensemble de données d'image, est enregistré de manière localisable, dans lequel des systèmes de coordonnées de référence ou des informations de direction sont en particulier déterminés ou planifiés dans l'ensemble de données.

4. Procédé selon l'une quelconque des revendications 2 ou 3, dans lequel le procédé d'imagerie est un procédé de tomographie assisté par ordinateur, un procédé de tomographie par résonance magnétique nucléaire ou un procédé de radiographie, en particulier avec une détection de volume.

5. Procédé selon l'une quelconque des revendications 1 à 4, dans lequel la planification de reconstruction s'effectue sur un système de navigation médical (15) qui dispose de l'ensemble de données de patient, en particulier de l'ensemble de données d'image et l'ensemble de données de modèle.

6. Procédé selon la revendication 5, dans lequel les régions de résection sont visualisées sur une sortie d'image (20) du système de navigation.

7. Procédé selon l'une quelconque des revendications 1 à 6, dans lequel une ou plusieurs des informations suivantes sont planifiées ou déterminées en tant que systèmes de coordonnées de référence ou en tant qu'informations de direction : l'axe de col fémoral (g), l'axe fémoral mécanique, le point central (M) de la tête fémorale (3), le rayon de la sphère de la tête fémorale (4), l'axe condylien postérieur, l'axe épicondylien, les plans pelviens, en particulier le plan pelvien médio-sagittal ou avant et le plan acétabulaire.

8. Procédé selon l'une quelconque des revendications 1 à 7, dans lequel l'attribution des données de modèle à l'ensemble de données de patient, en particulier à l'ensemble de données d'image s'effectue par une superposition assistée par ordinateur ou une mise en correspondance d'éléments des données de modèle et d'éléments de forme sensiblement correspondante provenant des données ou des données d'image de l'os d'articulation.

9. Procédé selon la revendication 1, dans lequel les données de modèle (4) elles-mêmes sont également prises en compte lors de la détermination des régions de résection (6) en tant que région partielle ou région partielle exclue.

10. Procédé selon l'une quelconque des revendications 1 à 9, dans lequel les données de modèle incluent des données de nature suivante :
- formes géométriques de base, en particulier des sphères, des corps creux sphériques, des cylindres, des formes de selle ou des combinaisons de ceux-ci,
- axes, surfaces, points centraux, circonférences et bords et leurs points ou surfaces,
- plages angulaires, en particulier avec les origines sur des points centraux, d'axe ou de surface prédéterminés ou caractéristiques,
- données de référence standardisées pour l'articulation, en particulier des données de modèle de référence,
ou sont composées de plusieurs des données susmentionnées.

11. Procédé selon la revendication 10, dans lequel les données de référence sont corrigées à l'aide de données typiques concernant une articulation ou un os d'articulation, par exemple des angles d'antétorsion, des angles de rétroversion, des angles de CCD, un emplacement de plan pelvien avant/sagittal, une inclinaison pelvienne.

12. Procédé selon la revendication 10 ou 11, dans lequel un modèle de données de référence adapté est sélectionné automatiquement à l'aide de l'ensemble de données de patient acquis.

13. Procédé selon l'une quelconque des revendications 1 à 12, lequel procédé est utilisé pour une articulation de la hanche, une articulation de l'épaule ou une articulation du genou.

14. Programme qui, lorsqu'il s'exécute sur un ordinateur ou lorsqu'il est chargé dans un ordinateur, amène l'ordinateur à mettre en oeuvre un procédé selon l'une quelconque des revendications 1 à 13.

15. Support de mémorisation de programme informatique comportant un programme selon la revendication 14.
